(19) Europäisches Patentamt

European Patent Office

Office européen des brevets

(11) **EP 3 442 442 B1**

(12) **FASCICULE DE BREVET EUROPEEN**

(45) Date de publication et mention de la délivrance du brevet:
**27.07.2022 Bulletin 2022/30**

(21) Numéro de dépôt: **17717716.9**

(22) Date de dépôt: **14.04.2017**

(51) Classification Internationale des Brevets (IPC):
**A61B 17/22** *(2006.01)*   **A61N 7/02** *(2006.01)*
**A61N 7/00** *(2006.01)*

(52) Classification Coopérative des Brevets (CPC):
**A61N 7/02; B06B 1/0655;** A61B 17/2202;
A61B 2017/22024; A61N 2007/0047;
A61N 2007/025

(86) Numéro de dépôt international:
**PCT/EP2017/059047**

(87) Numéro de publication internationale:
**WO 2017/178641 (19.10.2017 Gazette 2017/42)**

(54) **SONDE D'ABLATION THERMIQUE ULTRASONORE**

SONDE ZUR THERMISCHE ULTRASCHALLABLATION

ULTRASONIC THERMAL ABLATION PROBE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.04.2016   FR 1653377**

(43) Date de publication de la demande:
**20.02.2019   Bulletin 2019/08**

(73) Titulaire: **Carthera**
**75013 Paris (FR)**

(72) Inventeurs:
• **CANNEY, Michael**
**Denver**
**CO  80202 (US)**
• **BOUCHOUX, Guillaume**
**69100 Villeurbanne (FR)**
• **VIGNOT, Alexandre**
**69003 LYON (FR)**
• **LACOSTE, François**
**75014 PARIS (FR)**

(74) Mandataire: **Be IP**
**Cabinet LTL SAS**
**Centre d'Entreprise et d'Innovation**
**56, Bd Niels Bohr**
**CS 52132**
**69603 Villeurbanne Cedex (FR)**

(56) Documents cités:
EP-A2- 2 430 996        WO-A1-2011/053757
WO-A2-2011/146139        US-A1- 2006 273 695
US-A1- 2007 049 829

**Description**

## DOMAINE DE L'INVENTION

La présente invention concerne le domaine technique général du traitement de tissus par ultrasons, et en particulier le domaine technique des dispositifs d'émission d'ultrasons.

Plus précisément, la présente invention est relative aux sondes interstitielles pour le traitement d'une zone cible - telle qu'une tumeur maligne - par hyperthermie localisée à partir d'ondes ultrasonores de haute intensité.

## ARRIERE PLAN DE L'INVENTION

### 1. Généralités

Les ultrasons thérapeutiques sont aujourd'hui utilisés dans de nombreuses applications, notamment pour permettre l'ablation de tissus indésirables ou de tumeurs.

On a déjà proposé un dispositif de traitement ultrasonore par voie externe. Un tel dispositif comporte classiquement un (ou plusieurs) transducteur(s) pour la génération d'ultrasons focalisés de haute intensité vers une zone cible. Le (ou les) transducteur(s) ultrasonore(s) est (sont) adapté(s) pour concentrer une énergie acoustique en profondeur dans le corps du patient afin d'induire le chauffage et la destruction d'un tissu cible dans une approche non invasive.

On a également proposé un dispositif de traitement ultrasonore par voie interne destiné à être mis en contact avec la zone cible à traiter. Un tel dispositif de traitement par voie interne - connu sous le nom « *d'applicateur ultrasonore intra-tissulaire »* - permet le traitement de zones inaccessibles par voie externe. Ce dispositif comporte un (ou plusieurs) transducteur(s) monté(s) sur un support cylindrique de diamètre réduit (i.e. 2 à 3 mm) pour permettre leur application par voie interstitielle, ou au travers d'un cathéter.

Chaque transducteur peut présenter une surface plane, sphérique ou cylindrique et une forme généralement rectangulaire. Il permet l'application de puissances acoustiques comprises entre 1 et 50 W/cm$^2$ (préférentiellement entre 10 et 30W/cm$^2$) en générant des ondes ultrasonores divergentes ou légèrement convergentes au travers des tissus cibles.

L'efficacité du traitement par hyperthermie dépend du temps d'application des ondes ultrasonores par l'intermédiaire de l'applicateur intra-tissulaire. Un tissu est endommagé de façon permanente en moins d'une seconde si sa température atteint plus de 55 °C. Toute température supérieure à 41 °C peut endommager les tissus vivants, en fonction de la durée de l'hyperthermie. La notion de *«dose thermique* » est utilisée pour quantifier le risque de dommages aux tissus, et une dose thermique de plus de 240 minutes à 43°C est généralement considérée létale.

Afin de limiter le nombre de repositionnements de l'applicateur nécessaires au traitement d'un grand volume, il est généralement souhaitable de pouvoir chauffer le tissu aussi loin que possible (typiquement 10 à 30 mm) de l'applicateur.

Pour ce faire, il est nécessaire d'augmenter la puissance acoustique produite par le transducteur tout en limitant les risques de dégradation de celui-ci.

### 2. Solutions existantes pour augmenter l'efficacité de traitement

Pour augmenter la puissance acoustique produite par le transducteur d'applicateur intra-tissulaire, différentes solutions ont déjà été proposées.

### 2.1. Couche d'air sur la face arrière du transducteur

Par exemple, on a déjà proposé un applicateur incluant une couche d'air entre le transducteur et le support cylindrique du transducteur. Cette couche d'air permet de réduire la quantité d'énergie perdue lors de l'activation du transducteur en réfléchissant toute l'énergie acoustique générée par le transducteur en direction du support.

En effet, un transducteur est un élément piézoélectrique comprenant :

- une face avant s'étendant en regard de la zone cible à traiter, et
- une face arrière s'étendant en regard du support de l'applicateur.

Lors de l'activation du transducteur, celui-ci convertit l'énergie électrique en énergie mécanique et sa vibration génère une onde acoustique qui peut se propager vers l'avant et vers l'arrière du transducteur.

Une couche d'air en face arrière de l'élément piézoélectrique agit comme un miroir et réfléchit l'onde dirigée vers l'arrière du transducteur en direction de la face avant du transducteur. Ainsi, on évite de perdre une partie de l'énergie mécanique générée par le transducteur dans le milieu arrière.

Toutefois, un inconvénient de ce type d'applicateur concerne réchauffement du transducteur. En effet, le rendement électro-acoustique des éléments piézoélectriques est généralement de l'ordre de 60 à 70%. Ainsi, 30 à 40% de l'énergie électrique fournie à un transducteur piézoélectrique est dissipée sous forme de chaleur au travers du transducteur. Ceci induit une augmentation importante de la température au sein du transducteur.

Cet échauffement du transducteur peut induire sa dégradation. Par ailleurs, l'échauffement du transducteur peut réduire la profondeur de pénétration des ultrasons du fait de l'ébullition du milieu de propagation à proximité de la surface du transducteur. En effet, la présence de bulles de gaz empêche la propagation de l'énergie ultrasonore et peut provoquer la destruction indésirable de tissus sains collatéraux par diffusion thermique.

*2.2. Système de refroidissement sur la face arrière du transducteur*

Pour limiter les risques :

- de détérioration du transducteur, et
- de vaporisation des tissus au voisinage et/ou au contact du transducteur,

on a déjà proposé un applicateur intra-tissulaire incluant un système de refroidissement sur la face avant du transducteur.

On connaît également des applicateurs incluant un système de refroidissement sur la face arrière du transducteur pour permettre un meilleur contrôle de l'échauffement tissulaire en limitant les risques d'échauffement indésirable par conduction thermique du transducteur vers le tissu.

Toutefois, un inconvénient de la solution décrite ci-dessus est que la présence du système de refroidissement tend à réduire l'efficacité de traitement en profondeur dans une direction donnée. En effet, une partie de l'énergie acoustique générée par l'élément piézoélectrique est dirigée vers l'arrière du transducteur.

Le documents de brevet EP 2 430 996 A2, WO 2011/146139 A2, US 2006/273695 A1, WO 2011/053757 A1 et US 2007/049829 A1 sont des exemples de l'art antérieur pertinent.

*3. But de l'invention*

Un but de l'invention est de proposer une sonde interstitielle pour le traitement d'une zone cible par hyperthermie permettant de pallier au moins l'un des inconvénients précités.

Plus précisément, un but de la présente invention est de proposer une sonde interstitielle permettant générer des puissances acoustiques élevées par rapport à l'encombrement de la sonde, et d'éviter de surchauffer et vaporiser les tissus en contact avec la sonde, permettant des traitements plus rapides, plus efficaces et plus sûrs. Pour ce, on propose une sonde interstitielle :

- ayant un rendement de conversion électro-acoustique comparable à celui d'un applicateur intra-tissulaire incluant une couche d'air sur la face arrière du transducteur,
- comportant un système de refroidissement efficace et peu encombrant.

**BREVE DESCRIPTION DE L'INVENTION**

A cet effet, l'invention propose une sonde ultrasonore pour le chauffage, par voie interne, d'un milieu cible absorbant les ultrasons, la sonde comprenant :

- au moins un transducteur piézoélectrique comportant une face avant destinée à être positionnée en regard du milieu cible et une face arrière opposée à la face avant, le transducteur étant apte à émettre au moins une onde primaire émanant de sa face avant et au moins une onde secondaire émanant de sa face arrière,
- un réflecteur s'étendant en regard de la face arrière du transducteur, le réflecteur étant adapté pour réfléchir l'onde secondaire émise par le transducteur,
- une couche de fluide de refroidissement entre le transducteur et le réflecteur, remarquable en ce que l'épaisseur de la couche de fluide de refroidissement est adaptée en fonction :

  • du matériau constituant le fluide de refroidissement, et
  • de l'épaisseur et d'un matériau constituant le réflecteur, et
  • de la fréquence nominale du transducteur,

de sorte qu'une onde ultrasonore secondaire réfléchie par le réflecteur et se propageant vers la face avant interfère de façon constructive avec une onde ultrasonore primaire émanant de la face avant du transducteur.

Ainsi, l'invention offre la possibilité de disposer d'un refroidissement à l'aide d'un fluide de refroidissement - tel que de l'eau - sur la (ou les) face(s) arrière(s) du (ou des) transducteur(s) tout en conservant un rendement de conversion électro-acoustique aussi élevé que les applicateurs intra-tissulaires dont la face arrière du transducteur est refroidie par air.

Ceci permet d'obtenir une sonde interstitielle de traitement par hyperthermie dont l'efficacité est augmentée, ce qui autorise sa miniaturisation. Ce nouvel agencement permet de maximiser l'efficacité de la sonde en termes :

- de conversion électroacoustique d'une part, et
- de refroidissement des transducteurs d'autre part.

Les optimisations apportées à la sonde selon l'invention permettent d'accélérer la durée des traitements réalisés. En effet, le fait de refroidir les transducteurs avec un fluide de refroidissement permet d'augmenter les durées d'activation des transducteurs (notamment, il n'est plus nécessaire de désactiver les transducteurs pendant une certaine durée pour permettre leur refroidissement). De plus, un refroidissement efficace des transducteurs permet d'augmenter la puissance acoustique maximale que peut émettre la sonde, qui est principalement limitée par la surchauffe de l'élément piézoélectrique entraînant son altération. Une puissance acoustique accrue émise par la sonde permet de réduire le temps de traitement et de traiter un volume plus grand.

Des aspects préférés mais non limitatifs de la sonde de traitement selon l'invention sont les suivants :

- Le transducteur peut être alimenté électriquement par un signal électrique d'excitation induisant l'émission par ledit transducteur d'ondes ultrasonores à une fréquence nominale comprise entre 3 et 10 MHz, le réflecteur étant adapté pour réfléchir au moins 80% de l'énergie acoustique de l'onde secondaire émise par le transducteur à la fréquence nominale du transducteur, et le fluide de refroidissement ayant un coefficient d'atténuation acoustique inférieur à 1dB/cm à la fréquence nominale du transducteur, de préférence inférieur à 0.1 dB/cm à la fréquence nominale du transducteur ;
- En fonction de l'épaisseur et du matériau constituant le réflecteur, l'épaisseur de la couche de fluide de refroidissement peut être :

  • soit égale à un multiple impair du quart de la longueur d'onde de l'onde ultrasonore secondaire dans le matériau constitutif du fluide de refroidissement,
  • soit égale à un multiple entier de la moitié de la longueur d'onde de l'onde ultrasonore secondaire dans le matériau constitutif du fluide de refroidissement.

- Dans une variante de réalisation de la sonde :

  • l'impédance acoustique du matériau constituant le réflecteur est supérieure à $10^7$ kg/(m$^2$s), et
  • l'épaisseur de la couche de fluide de refroidissement est égale à un multiple impair du quart de la longueur d'onde de l'onde ultrasonore secondaire dans le matériau constitutif du fluide de refroidissement.

- L'épaisseur du réflecteur peut être supérieure ou égale à 20 μm, préférentiellement supérieure à 30 μm, et encore plus préférentiellement supérieure à 40 μm.
- Le réflecteur peut comprendre une paroi interne incluant une première face en regard de la face arrière du transducteur et une deuxième face opposée, la deuxième face étant en contact avec de l'air ;
- Dans une variante de réalisation de la sonde :

  • l'impédance acoustique du matériau constituant la paroi interne est comprise entre $1{\times}10^6$ kg/(m$^2$s) et $10{\times}10^6$kg/(m$^2$s),
  • l'épaisseur de la paroi interne est égale à un multiple impair du quart de la longueur d'onde de l'onde ultrasonore secondaire dans le matériau constituant la paroi interne, et
  • l'épaisseur de la couche de fluide de refroidissement est égale à un multiple impair du quart de la longueur d'onde de l'onde ultrasonore secondaire dans le fluide de refroidissement ;

- Dans une variante de réalisation de la sonde :

  • l'impédance acoustique du matériau constituant la paroi interne est comprise entre $1{\times}10^6$ kg/(m$^2$s) et $10{\times}10^6$kg/(m$^2$s), et
  • l'épaisseur de la paroi interne et l'épaisseur de la couche de fluide de refroidissement ne sont pas comprises dans des plages de plus ou moins 25% autour d'un multiple entier de la moitié de la longueur d'onde de l'onde ultrasonore secondaire dans le matériau constituant la paroi interne ;

- Dans une variante de réalisation de la sonde :

  • l'impédance acoustique du matériau constituant la paroi interne est comprise entre $1 \times 10^6$ kg/(m$^2$s) et $10 \times 10^6$ kg/(m$^2$s),
  • l'épaisseur de la paroi interne est égale à un multiple entier de la moitié de la longueur d'onde de l'onde ultrasonore secondaire dans le matériau constituant la paroi interne,
  • l'épaisseur de la couche de fluide de refroidissement est égale à un multiple entier de la moitié de la longueur d'onde de l'onde ultrasonore secondaire dans le fluide de refroidissement ;

- Dans une variante de réalisation de la sonde :

  • l'impédance acoustique du matériau constituant la paroi interne est comprise entre $1 \times 10^6$ kg/(m$^2$s) et $10 \times 10^6$ kg/(m$^2$s), et
  • l'épaisseur de la paroi interne et l'épaisseur de la couche de fluide de refroidissement ne sont pas comprises dans des plages de plus ou moins 25% autour d'un multiple impair du quart de la longueur d'onde de l'onde ultrasonore secondaire dans le matériau constituant la paroi interne.

- La sonde peut comprendre en outre une couche d'adaptation - par exemple composée de parylène - sur la face avant du transducteur, ladite couche d'adaptation ayant une épaisseur égale à un multiple du quart de la longueur d'onde de l'onde ultrasonore dans le matériau constitutif de ladite couche d'adaptation - par exemple sensiblement égale à 80 $\mu$m pour une fréquence centrale de résonance égale à 6 MHz.

L'optimisation de l'épaisseur de la couche de fluide de refroidissement permet d'une part de limiter l'encombrement de la sonde, et d'autre part d'améliorer le rendement des transducteurs, et donc d'augmenter la puissance acoustique qu'ils peuvent émettre pour accélérer le traitement et accroître la profondeur de pénétration.

## BREVE DESCRIPTION DES DESSINS

D'autres avantages et caractéristiques de la sonde selon l'invention ressortiront mieux de la description qui va suivre de plusieurs variantes d'exécution, données à titre d'exemples non limitatifs, à partir des dessins annexés sur lesquels :

- la figure 1 illustre schématiquement un exemple de sonde de traitement par hyperthermie d'une zone cible,
- la figure 2 illustre un système complet incluant la sonde de traitement,
- les figures 3 à 5 sont des vues en coupe transversale de différents modes de réalisation d'une partie active d'une sonde de traitement,
- les figures 6A à 6C sont des courbes illustrant l'efficacité d'une sonde de traitement,
- la figure 7 est une représentation schématique de différentes étapes d'un procédé de conception d'une sonde de traitement.

## DESCRIPTION DETAILLEE DE L'INVENTION

### 1. Généralités

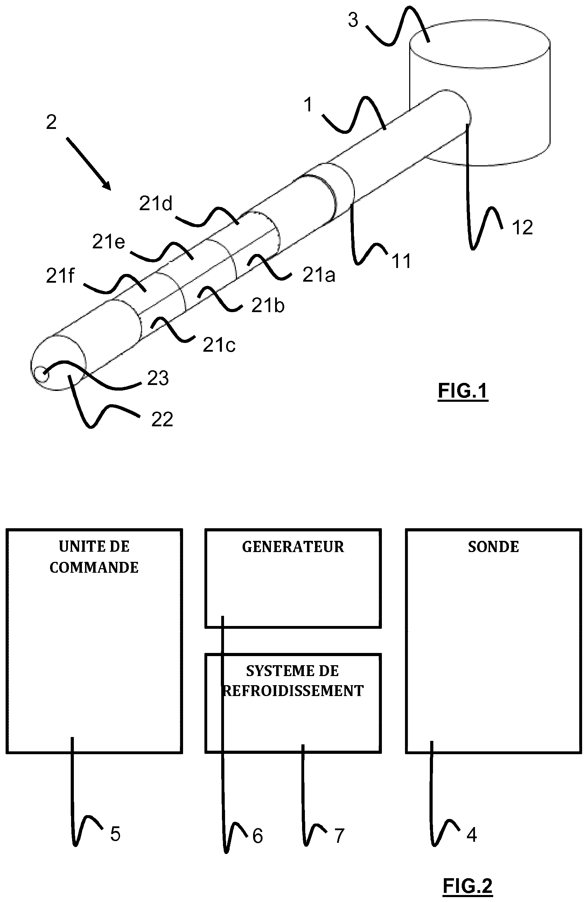

En référence à la figure 1, on a illustré un exemple de sonde de traitement par hyperthermie d'une zone cible. La sonde - ou applicateur- est destinée à être introduite dans le corps d'un patient pour permettre le traitement de la zone cible. Le diamètre de la sonde est de préférence inférieur à 5 mm (notamment égal à 3.5 mm) pour faciliter son introduction à l'intérieur du patient, par voie interstitielle ou au travers d'un cathéter.

La sonde comprend :

- un corps longitudinal flexible 1 de forme générale cylindrique,
- une partie active 2 montée à une extrémité distale 11 du corps 1, la partie active 2 incluant un (ou plusieurs) transducteur(s) ultrasonore(s) 21a-21f,
- un boîtier de connexion 3 monté à une extrémité proximale 12 du corps 1 pour raccorder électriquement la partie active à une unité de commande distante permettant le contrôle du (ou des) transducteur(s) 21a-21f.

### 1.1. *Corps*

Le corps 1 peut être composé d'une gaine souple réalisée dans un matériau choisi pour ses caractéristiques de non toxicité et de bonne tolérance.

Le corps 1 peut comprendre un ou plusieurs canaux, éventuellement coaxiaux, pour le passage :

- d'un (ou plusieurs) câble(s) électrique(s) pour connecter électriquement le(s) transducteur(s) à l'unité de commande 5,
- d'un fluide de refroidissement, et/ou
- d'un outil de chirurgical permettant de réaliser une biopsie, etc.

La structure du corps 1 étant connue en elle-même de l'homme du métier, celle-ci ne sera pas décrite plus en détails dans la suite.

### 1.2. *Unité de commande 5*

En référence à la figure 2, l'unité de commande 5 permet de contrôler le(s) transducteur(s) ultrasonore(s) 21a-21f, et éventuellement de traiter des signaux reçus de détecteurs montés sur la partie active 2 de la sonde 4.

L'unité de commande 5 est connectée à un générateur 6 pour alimenter électriquement les transducteurs 21a-21f et des moyens de saisie pour spécifier les paramètres - tels que la fréquence et/ou la puissance, etc. - du courant électrique d'alimentation du (des) transducteur(s) 21a-21f.

L'unité de commande 5 est également reliée à un système de refroidissement 7 pour l'alimentation en fluide de refroidissement de la partie active 2 de la sonde 4.

Les éléments constitutifs de l'unité de commande 5 peuvent être réalisés d'une manière programmée ou câblée. Les différents circuits constituant l'unité de commande 5 sont connus en soi et ne seront pas décrits plus précisément.

### 1.3. *Partie active 2*

La partie active 2 est de forme générale cylindrique.

Elle comprend une tête 22 de forme sphérique ou conique pour faciliter l'insertion de la sonde dans le corps du patient. La tête 22 peut présenter une lumière traversante 23 pour le passage d'un outil de biopsie.

La partie active 2 comprend également :

- un (ou des) transducteur(s) ultrasonore(s) 21a-21f monté(s) sur sa paroi latérale,
- un (ou plusieurs) réflecteur(s) acoustique(s) monté(s) en regard d'une (ou de) face(s) arrière(s) du (ou des) transducteur(s) 21a-21f, et
- un fluide de refroidissement s'écoulant entre le (ou les) réflecteur(s) et le (ou les) transducteur(s).

La présence combinée :

- d'un réflecteur sur la face arrière de chaque transducteur, et
- d'un fluide de refroidissement entre les réflecteur(s) et transducteur(s)

permet d'augmenter l'efficacité de traitement de la sonde tout en assurant son refroidissement.

En effet, chaque transducteur 21a-21f comprend une face avant destinée à faire face à la zone cible à traiter et une face arrière opposée à la face avant. Lors de l'activation du transducteur, celui-ci convertit l'énergie électrique qui lui est fournie :

- en ondes acoustiques primaires se propageant vers l'extérieur de la partie active (i.e. propagation vers l'avant du transducteur), et
- en ondes secondaires se propageant vers l'intérieur de la partie active (i.e. propagation vers l'arrière du transducteur).

La présence d'un réflecteur acoustique disposé en regard de la face arrière de chaque transducteur permet de réfléchir les ondes ultrasonores secondaires vers l'extérieur de la partie active (i.e. en direction de la zone cible). Ainsi ces ondes ultrasonores secondaires réfléchies se combinent aux ondes ultrasonores primaires, ce qui permet d'augmenter la quantité d'énergie acoustique utile au traitement de la zone cible.

En outre, la présence d'un fluide de refroidissement permet de limiter réchauffement local de chaque transducteur, ce qui réduit les risques de formation de bulles de gaz susceptibles d'empêcher la propagation de l'énergie ultrasonore générée par chaque transducteur.

On va maintenant décrire plus en détails les caractéristiques des différents éléments constituant la partie active 2.

### 1.3.1. *Transducteur 21*

Chaque transducteur 21a-21f peut être plan, convergent (concave), ou divergent (convexe).

Les transducteurs plans, légèrement focalisés ou divergents présentent l'avantage d'être particulièrement adaptés à une application interstitielle pour amener le transducteur au plus près du tissu cible à traiter.

Chaque transducteur 21a-21f peut être composé d'un élément piézoélectrique de forme rectangulaire (transducteur plan), cylindrique ou en portion de cylindre (transducteur divergent). La fréquence de résonance nominale de chaque transducteur 21a-21f est comprise entre 250kHz et 21 MHz, de préférence comprise entre 3 à 10 MHz (et encore plus préférentiellement entre 4 et 6 MHz). La puissance acoustique émise par ces types de transducteur est de quelques dizaines de Watt par centimètre carré (W/cm$^2$).

Dans la suite de la description, on décrira plus précisément l'invention en référence à l'utilisation d'un (ou plusieurs) transducteur(s) divergent(s), étant bien entendu que l'invention pourrait s'appliquer à l'utilisation d'un (ou plusieurs) transducteur(s) plan(s).

La partie active 2 peut comprendre un unique transducteur monobloc tubulaire. L'utilisation d'un transducteur tubulaire permet de faciliter la fabrication de la sonde de traitement, ce type de transducteur étant résistant et facile à coller et à souder. Le transducteur comprend :

- une électrode intérieure formant face arrière (i.e. la face interne du tube) et
- une électrode extérieure formant face avant du transducteur tubulaire (i.e. la face externe du tube).

En variante la partie active peut comprendre une pluralité de transducteurs distincts, chaque transducteur ayant une forme de portion de cylindre, les transducteurs étant disposés les uns par rapport aux autres pour former un ensemble tubulaire. Cet ensemble de transducteurs peut être obtenu par sectorisation d'un transducteur monobloc tubulaire. La méthode de sectorisation peut consister à sectionner l'électrode extérieure du transducteur monobloc tubulaire selon sa hauteur et/ou sa circonférence.

Par exemple dans le mode de réalisation illustré à la figure 1, la partie active 2 comprend trois motifs annulaires de transducteurs disposés le long de l'axe longitudinal de la partie active 2, chaque motif annulaire étant composé de quatre transducteurs en quart de cylindre disposés radialement autour de la paroi latérale de la partie active 2.

### 1.3.2. *Réflecteur 24*

Le réflecteur acoustique permet de réfléchir les ondes ultrasonores secondaires se propageant depuis la face arrière de chaque transducteur.

Le réflecteur est de préférence tubulaire et comprend :

- une face extérieure convexe en regard de la face arrière du transducteur, et
- une face intérieure concave opposée à la face extérieure.

Le réflecteur permet de réfléchir les ondes ultrasonores se propageant vers l'arrière en direction de la face avant du transducteur. Le matériau constituant le réflecteur dépend de la structure générale de la sonde.

Par exemple dans certains modes de réalisation, le réflecteur est un matériau de faible impédance acoustique, tel qu'un ensemble composé d'un gaz et d'une couche de polyétheréthercétone (ci-après désigné *« PEEK »*, acronyme de l'expression anglosaxonne *« PolyEtherEtherKetone »).*

Dans d'autres modes de réalisation, le réflecteur comprend une couche de matériau de haute impédance acoustique, tel que du métal (laiton, etc.) ou de la céramique.

Le réflecteur est de préférence choisi de sorte à réfléchir au moins 90% de l'énergie acoustique incidente à la fréquence nominale de chaque transducteur, et de préférence au moins 95% de l'énergie acoustique incidente à la fréquence nominale de chaque transducteur.

### 1.3.3. *Fluide de refroidissement 25*

Le fluide de refroidissement permet de limiter l'échauffement de chaque transducteur. Le fluide de refroidissement peut être un liquide (tel que de l'eau), un gel caloporteur, etc.

Avantageusement, le fluide de refroidissement présente une atténuation acoustique inférieure à un décibel par centimètre (1 dB/cm) à la fréquence nominale de chaque transducteur, et préférentiellement inférieure à un dixième de décibel par centimètre (0.1 dB/cm). Ceci permet de maximiser la quantité d'énergie acoustique redirigée par le réflecteur vers l'extérieur de la sonde.

### 1.3.4. Dimensionnement

Avantageusement, les épaisseurs :

- du réflecteur d'une part, et
- de la couche de fluide de refroidissement d'autre part

peuvent être choisies pour maximiser le coefficient de réflexion équivalent en face arrière de l'élément piézoélectrique à la fréquence de fonctionnement. Pour ce, les épaisseurs peuvent être choisies de sorte que les ondes ultrasonores secondaires réfléchies par le réflecteur et se propageant vers la face avant interfèrent de façon constructive avec les ondes ultrasonores primaires émanant de la face avant du transducteur et se propageant vers l'extérieur de la sonde.

Plus précisément, les dimensions de la couche de fluide et du réflecteur acoustiques sont choisies de sorte que les ondes primaires et secondaires soient en phase au niveau de la face avant de chaque transducteur afin de créer un effet d'amplification des ondes acoustiques se propageant vers l'extérieur de la sonde à la fréquence de fonctionnement.

Ceci permet de maximiser l'énergie acoustique émise vers la zone cible, et donc d'améliorer l'efficacité de la sonde de traitement.

Comme il ressortira plus clairement de la description qui va suivre, les épaisseurs du réflecteur et de la couche de fluide sont choisies en fonction :

- du matériau constituant le fluide de refroidissement,
- du matériau constituant la paroi interne,
- de la fréquence nominale de chaque transducteur.

### 2. Principe

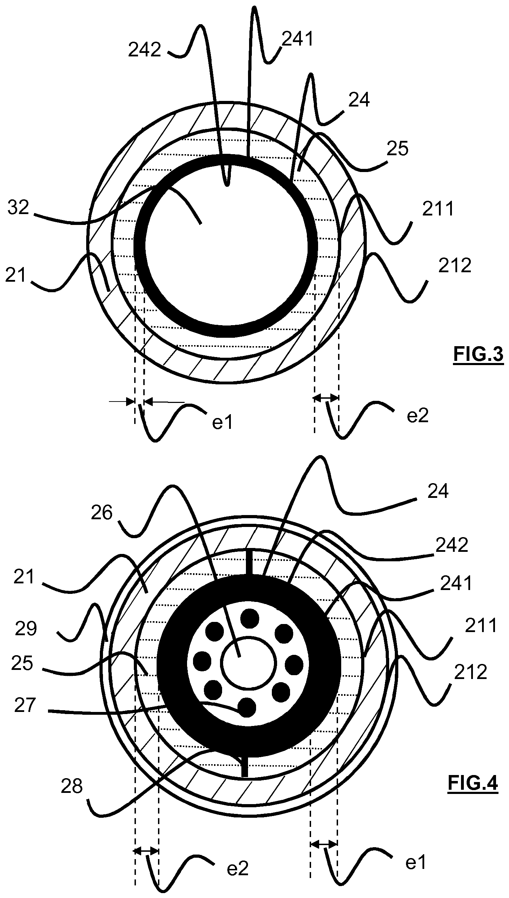

En référence à la figure 3, on a illustré la structure de base de la partie active 2.

La partie active 2 comporte un transducteur monobloc tubulaire unique 21, une paroi interne tubulaire 24, un fluide de refroidissement 25 entre le transducteur 21 et la paroi interne 24.

Le transducteur 21 comprend une électrode intérieure formant une face arrière 211 (i.e. la face interne du tube) reliée à la masse et une électrode extérieure formant une face avant 212 (i.e. la face externe du tube). Le transducteur 21 a un diamètre de 3.5 mm et une hauteur de 10 à 40 mm.

La paroi interne tubulaire 24 s'étend à l'intérieur du transducteur tubulaire 21. La paroi interne 24 comprend une première face 241 en regard de la face arrière 211 du transducteur 21 et une deuxième face 242 opposée. Avantageusement, la paroi interne 24 est positionnée sur la partie active 2 de sorte que les axes de révolution du transducteur 21 et de la paroi interne 24 sont coaxiaux. La paroi interne 24 peut être réalisée dans divers matériaux. Par exemple un matériau de forte impédance tel que l'alumine ou un métal tel que le cuivre ou l'acier.

Elle peut être aussi réalisée dans un matériau de relativement faible impédance - tel que du PEEK - d'impédance acoustique comprise entre $1 \times 10^6$ kg/(m$^2$s) et $10 \times 10^6$ kg/(m$^2$s). Dans ce cas le matériau 32 situé à l'intérieur de la paroi 24 doit comporter une très faible impédance, par exemple de l'air ou un gaz, de façon à ce que l'ensemble paroi 24 - matériau 32 se comporte en réflecteur des ondes arrière.

La paroi interne 24 présente une épaisseur e1.

Le fluide de refroidissement 25 est un fluide caloporteur situé ou s'écoulant entre la face arrière du transducteur 21 et la première face de la paroi interne 24. Dans le mode de réalisation illustré à la figure 3, le fluide de refroidissement 25 est constitué d'une couche d'eau d'épaisseur e2.

La présence du fluide de refroidissement à l'intérieur de la partie active 2 permet d'évacuer ou d'emmagasiner la chaleur générée au niveau du transducteur 21.

Avantageusement, les épaisseurs e1, e2 de la paroi interne 24 et de la couche 25 de fluide de refroidissement peuvent être optimisées de sorte que l'énergie acoustique réfléchie par le réflecteur arrive en phase à la surface du transducteur 21.

### 2.1. Cas d'un réflecteur de faible impédance acoustique (ensemble PEEK-air):

#### 2.1.1. Configuration 1

Lorsque le réflecteur présente une faible impédance acoustique (par exemple réflecteur composé d'un gaz et d'une couche de PEEK), l'épaisseur e1 de la paroi interne 24 peut être choisie égale à un multiple impair du quart de la longueur d'onde dans le matériau de la paroi interne - afin de maximiser la réflexion de l'onde de pression dans le fluide de refroidissement 25 sur la paroi externe de 24, à la fréquence de fonctionnement.

Pour que l'onde acoustique réfléchie par le réflecteur arrive en phase avec l'onde se propageant vers l'avant à la surface du transducteur 21, l'épaisseur e2 de la couche 25 de fluide de refroidissement (correspondant à la distance entre la paroi interne et le transducteur) peut aussi être choisie égale à un multiple impair du quart de la longueur d'onde de l'onde ultrasonore secondaire dans le fluide de refroidissement.

Typiquement, si le fluide de refroidissement est de l'eau, que la fréquence nominale du transducteur est de 6MHz, et que le réflecteur est composé d'un gaz et d'une couche de PEEK, alors l'épaisseur e2 de la couche 25 de fluide de refroidissement peut être égale à 63 $\mu$m, 189 $\mu$m, 315 $\mu$m, etc., et l'épaisseur du tube interne en PEEK peut être de 108 $\mu$m, 324 $\mu$m, 540 $\mu$m...

#### 2.1.2. Configuration 1bis

En variante, les épaisseurs e1 et e2 peuvent être choisies égales à un multiple de demilongueur d'onde dans le matériau correspondant.

Typiquement, si le fluide de refroidissement est de l'eau et que la fréquence nominale du transducteur 21 est de 6MHz, alors l'épaisseur e2 de la couche de fluide de refroidissement 25 peut être égale à 125 $\mu$m, 250 $\mu$m, 375 $\mu$m, etc.

L'optimisation de l'épaisseur de la couche 25 de fluide de refroidissement conduit à une efficacité accrue de la sonde de traitement qui peut approcher l'efficacité d'une sonde ayant une couche d'air sur la face arrière 211 du transducteur 21.

### 2.2. Cas d'un réflecteur de haute impédance acoustique (configuration 2)

Lorsque le réflecteur comprend une couche de matériau de haute impédance acoustique - c'est-à-dire une impédance acoustique supérieure à $10^7$ kg/(m²s) tel que de l'alumine ou du laiton ou acier inox - l'épaisseur e1 peut être choisie égale à un multiple impair du quart de la longueur d'onde dans le matériau de la paroi interne - afin de maximiser la réflexion de l'onde de pression dans le fluide de refroidissement 25 sur la paroi externe de 24, à la fréquence de fonctionnement.

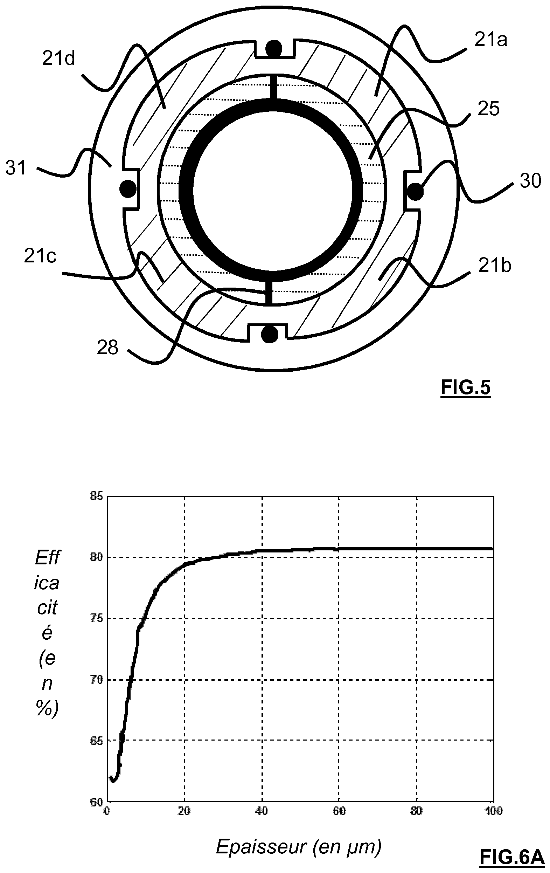

Cette épaisseur dépend du matériau de la paroi interne 24. Par exemple si le matériau de la paroi interne 24 est du cuivre l'épaisseur doit être d'au moins 20 microns (figure 6A).

En effet, comme représenté sur la figure 6A qui illustre l'efficacité du transducteur en fonction de l'épaisseur e1 du réflecteur pour une fréquence nominale du transducteur égale à 6 MHz, l'efficacité du transducteur est maximale lorsque l'épaisseur du réflecteur est supérieure à 20 $\mu$m. Ceci s'explique par le fait que lorsque l'épaisseur du réflecteur est supérieure à 20 $\mu$m, celui-ci réfléchit la totalité des ondes acoustiques secondaire émanant de la face arrière du transducteur.

Pour que l'énergie acoustique réfléchie par le réflecteur arrive en phase à la surface du transducteur, l'épaisseur e2 de la couche de fluide de refroidissement (correspondant à la distance entre la paroi interne et le transducteur) est choisie égale à un multiple impair du quart de la longueur d'onde de l'onde ultrasonore secondaire dans le fluide de refroidissement.

Typiquement, si le fluide de refroidissement est de l'eau et que la fréquence nominale du transducteur est de 6MHz, alors l'épaisseur de la couche de fluide de refroidissement peut être égale à 63 $\mu$m, 189 $\mu$m, 315 $\mu$m, etc.

L'optimisation de l'épaisseur e2 de la couche 25 de fluide de refroidissement conduit à une efficacité accrue de la sonde de traitement à la fréquence de fonctionnement.

### 2.3. *Optimisation des épaisseurs du réflecteur et de la couche de fluide de refroidissement*

Dans chacune des configurations décrites précédemment, le transducteur est refroidi en utilisant un fluide de refroidissement dont l'épaisseur e2 est optimisée de sorte que l'onde réfléchie par le réflecteur arrive en phase sur le transducteur. Ceci permet d'optimiser l'efficacité de l'émission acoustique.

Il en va de même de l'épaisseur e1 du réflecteur qui est choisie de sorte que les ondes acoustiques secondaires réfléchies par le réflecteur arrivent en phase à la surface du transducteur, ce qui conduit à une efficacité accrue de la sonde de traitement.

Le tableau ci-dessous illustre des exemples d'épaisseurs e1, e2 optimisées pour les différentes configurations 1, 2 et 1bis de la sonde de traitement, à 6 MHz.

| configuration | Epaisseur e2 de la couche de fluide de refroidissement dans le cas de l'eau | Epaisseur e1 du tube interne | Matériau constituant le tube interne | Matériau en regard de la 2ème face du tube interne | Efficacité Maximale à une Fréquence nominale de 6MHz |
|---|---|---|---|---|---|
| 1 | $(2N+1)\frac{\lambda_{eau}}{4}$ <br> **189 μm** | $(2N+1)\frac{\lambda}{4}$ <br> **108 μm** | PEEK $\lambda$ = 431 μm $Z$ = 3.23 $MR$ | Air | 81% |
| 2 | $(2N+1)\frac{\lambda_{eau}}{4}$ <br> **189 μm** | $(2N+1)\frac{\lambda}{4}$ <br> **417 μm (N=0)** | Alumine $\lambda$ = 1667 μm $Z$ = 39.8 $MR$ | Eau ou air | 81% |
| 1 bis | $N\frac{\lambda_{eau}}{2}$ <br> **125 μm** | $N\frac{\lambda}{2}$ <br> **216 μm** | PEEK $\lambda$ = 431 μm $Z$ = 3.23 $MR$ | Air | 79% |

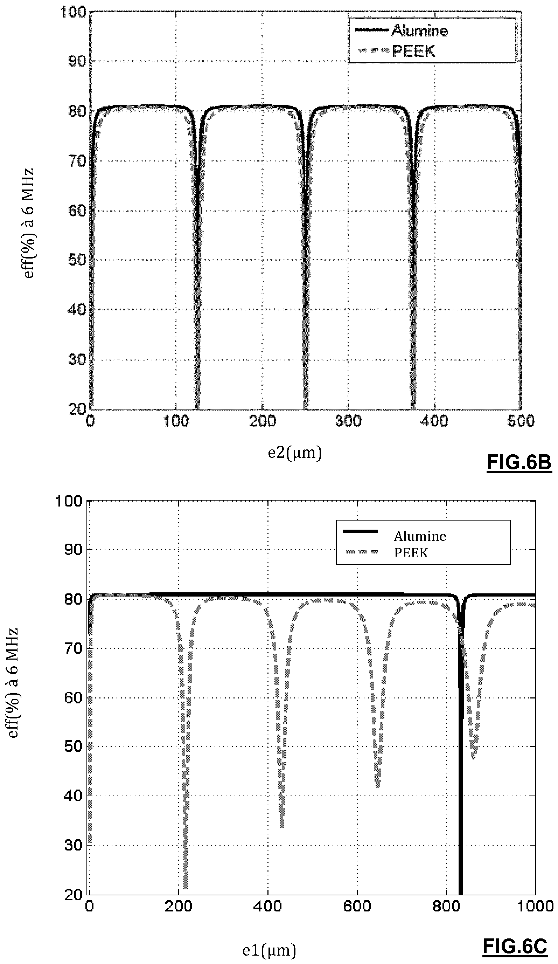

En pratique on peut s'éloigner des épaisseurs préconisées dans ce tableau et garder un bon rendement, Il faut surtout noter les épaisseurs « interdites » pour lesquelles l'efficacité est faible (figures 6)

Les épaisseurs préconisées dans ce tableau ne sont qu'indicatives et peuvent être optimisées expérimentalement (ce n'est qu'un modèle simple qui est utilisé pour ce tableau, et les paramètres des matériaux utilisés par ce modèle ne sont qu'indicatifs)

La figure 6B illustre la réflectivité calculée en fonction de e1 pour les configurations 1 et 2. Elle couvre une plage d'épaisseurs plus importante, ce qui fait ressortir la baisse de réflectivité vers 840 microns (config 2, alumine). Noter qu'elle est obtenue avec une épaisseur e2 de la couche de fluide de refroidissement optimale de 189 μm (3*lambda/4 comme dans le tableau, mais sinon 63μm c'est à dire lambda/4, donnerait les mêmes courbes)

La figure 6C illustre la réflectivité calculée en fonction de e2 également pour les configurations 1 et 2, e1 étant fixé à l'optimal (suivant valeur du tableau). L'épaisseur e1 du réflecteur alumine est lambda/4=417 μm (courbe noire), et l'épaisseur e1 du réflecteur PEEK (courbe gris tirets) est lambda/4=108 μm

### 3. *Caractéristiques optionnelles*

On va maintenant décrire des caractéristiques optionnelles de l'invention. Il est bien entendu que l'invention ne se limite pas à une sonde incluant ces caractéristiques optionnelles.

En référence à la figure 4, on a illustré un mode de réalisation de la partie active 2. Le transducteur 21 et le réflecteur 24 sont du même type que dans l'exemple illustré à la figure 3 et ne seront pas décrits plus en détails.

Dans ce mode de réalisation, le canal central 26 constitue un canal de biopsie pour le prélèvement éventuel d'une partie d'organe ou d'un tissu afin d'effectuer des examens. Le lecteur appréciera que le canal de biopsie peut être constitué par le tube 24 lui-même, sans ajout d'un canal central 26 supplémentaire.

La circulation du fluide de refroidissement est assurée par la conduite définie entre la face arrière 211 du transducteur 21 et la première face 241 de la paroi interne 24. Plus précisément, la conduite comprend deux cloisons 28 de séparation s'étendant radialement et permettant de subdiviser la conduite en deux chambres distinctes :

- l'une des chambres permet l'amené de fluide de refroidissement, et
- l'autre des chambres permet l'évacuation du fluide de refroidissement.

Avantageusement, ces deux chambres communiquent à leurs extrémités distales et sont reliées à des pompes et des réservoirs pour permettre la circulation du fluide de refroidissement.

La partie active 2 peut également comprendre une (ou plusieurs) couche(s) d'adaptation d'impédance acoustique 29 recouvrant la face avant 212 du transducteur 21. La couche d'adaptation 29 est réalisée dans un matériau - tel que du parylène - dont l'impédance acoustique est comprise entre l'impédance du transducteur piézoélectrique 21 et l'impédance acoustique de la zone cible. La présence d'une couche d'adaptation 29 permet de limiter les réflexions d'ondes ultrasonores à l'interface entre le transducteur 21 et le milieu extérieur de sorte à transférer un maximum d'énergie acoustique vers la zone cible. Elle permet en outre d'isoler électriquement le transducteur 21. L'épaisseur de la couche d'adaptation 29 est de préférence égale à un multiple entier du quart de la longueur d'onde (dans le matériau constituant la couche d'adaptation) à la fréquence nominale du transducteur 21. Les caractéristiques d'une telle couche d'adaptation 29 sont connues de l'homme du métier et ne seront pas décrites plus en détails dans la suite.

Avantageusement, le canal central 26 et la paroi interne 24 sont coaxiaux. Ceci permet d'équilibrer la partie active 2. Toutefois dans d'autres modes de réalisation, le canal central 26 et la paroi interne 24 peuvent ne pas être coaxiaux, le canal central 26 s'étendant à l'intérieur de la paroi interne 24.

La paroi interne 24 et le canal central 26 définissent un espace libre à l'intérieur duquel des fils électriquement conducteurs 27 peuvent être positionnés pour relier électriquement le transducteur 21 à l'unité de commande 5. Ceci permet d'alimenter le transducteur 21 en énergie électrique. En variante, le transducteur 21 peut être alimenté électriquement par l'extérieur, par exemple en utilisant un circuit imprimé souple de connexion enroulé autour de la face avant 212 du transducteur 21.

Outre des fils électriques 27, l'espace libre entre le canal central 26 et la paroi interne 24 peut contenir un matériau d'impédance acoustique très différente de celle du fluide de refroidissement - tel que de l'air ou une mousse expansée. Ceci permet de garantir qu'au moins 90% de l'énergie acoustique incidente se propageant vers la paroi interne 24 soit réfléchie vers le transducteur 21.

En référence à la figure 5, on a illustré un autre mode de réalisation de la partie active.

Ce mode de réalisation diffère du mode de réalisation illustré à la figure 4 en ce que le transducteur 21 est composé de quatre ensembles d'éléments piézoélectriques 21a-21d obtenus en sectorisant un transducteur tubulaire monobloc à l'aide d'un laser haute puissance ou d'un fraisage mécanique permettant de former des rainures longitudinales. Ces rainures longitudinales peuvent ensuite être utilisées pour le passage de câbles 30 de connexion des différents éléments piézoélectriques 21a-21d. Chaque ensemble peut comprendre six à huit (ou plus) élément piézoélectriques dans le sens de la longueur. Avantageusement, les différents éléments piézo-électriques (au nombre de 24 ou 32) peuvent être commandés indépendamment. Ceci permet d'ajuster le traitement en fonction de la taille de la tumeur - et notamment de son extension longitudinale.

Les rainures longitudinales (en réalité la partie non émissive des transducteurs) doivent être assez fines de façon à ne pas réduire le diagramme de rayonnement acoustique des éléments. Si c'était le cas, on obtiendrait un diagramme en « pétales » plutôt que circulaire. A titre indicatif, la largeur des rainures doit être inférieure à la longueur d'onde dans le milieu de propagation avant, et la somme de la largeur des rainures doit être inférieure au huitième du périmètre de l'extérieur de la sonde. Par exemple la largeur des rainures doit être inférieure à 300 microns pour un transducteur en 4 éléments de diamètre extérieur 3.5mm fonctionnant à 4MHz.

La partie active comprend également un ballonnet 31 à volume variable s'étendant sur la face extérieur du transducteur 21.

Le ballonnet 31 est connecté à des moyens d'alimentation en fluide (gaz ou liquide) permettant de faire varier son volume entre :

- une conformation rétractée où le volume du ballonnet est minimale, et
- une conformation déployée (telle qu'illustrée sur la figure 5) où le volume du ballonnet est maximale.

Par exemple, le ballonnet peut être connecté au système de refroidissement 7 pour l'alimentation en fluide de refroidissement 25 de la partie active 2. Dans ce cas, le ballonnet constitue un conduit d'acheminement du fluide de refroidissement : le système de refroidissement 7 alimente l'intérieur de la partie active 2 en fluide de refroidissement et celui-ci est retourné au système de refroidissement 7 par l'intermédiaire du ballonnet 31.

La présence d'un ballonnet 31 présente de nombreux avantages. Notamment, le ballonnet 31 permet de refroidir le tissu à proximité de la sonde ainsi que la face avant du transducteur. Le ballonnet forme également un moyen d'isolation électrique de la sonde.

## 4. *Procédé de fabrication*

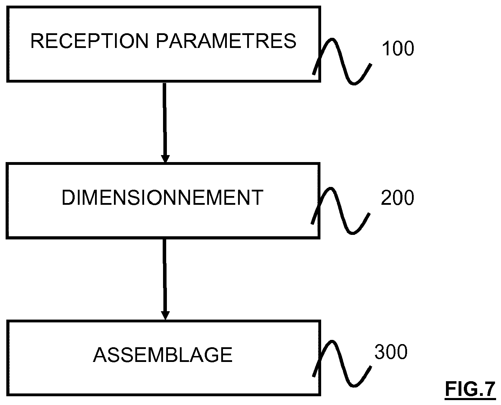

En référence à la figure 7, on a illustré un exemple de procédé de conception d'une sonde ultrasonore pour le chauffage d'un milieu cible.

Le procédé comprend une étape 100 de réception de paramètres de fonctionnement désirés pour la sonde. Ces paramètres dépendent notamment de l'application visée pour la sonde de traitement. Ces paramètres comprennent de façon non exhaustive :

- La fréquence nominale d'utilisation du transducteur,
- La présence ou non d'un canal de biopsie,
- Le type de matériau désiré pour le réflecteur,
- Le type de matériau situé sur la deuxième face du réflecteur,
- Le type de fluide de refroidissement utilisé, etc.

Le procédé comprend ensuite une étape 200 de dimensionnement de la sonde. Cette étape consiste notamment à définir une épaisseur optimale de la couche de fluide de refroidissement de sorte qu'une onde ultrasonore secondaire émanant de la face arrière du transducteur et se propageant vers la face avant interfère de façon constructive avec une onde ultrasonore primaire émanant de la face avant du transducteur, l'épaisseur de ladite couche de fluide de refroidissement étant calculée en fonction :

- du matériau constituant le fluide de refroidissement,
- de l'épaisseur et du matériau constituant la paroi interne,
- de la fréquence centrale de résonance.

Le procédé comprend enfin une étape 300 d'assemblage des différents éléments constituant la sonde.

*5. Conclusions*

La combinaison d'un réflecteur et d'un fluide de refroidissement sur la face arrière du transducteur permet d'obtenir une sonde de traitement intra-tissulaire :

- dont l'efficacité est proche de celle d'une sonde d'ablation thermique ultrasonore dont la face arrière du transducteur est recouverte d'une couche d'air,
- tout en limitant l'augmentation locale de température du transducteur.

Le lecteur aura compris que de nombreuses modifications peuvent être apportées à l'invention décrite précédemment sans sortir matériellement des nouveaux enseignements et des avantages décrits ici.
Notamment, l'épaisseur optimisée e2 de la couche de fluide et celle e1 du tube interne peuvent être assez proche (+/- 25%) - sans être rigoureusement égale - à un multiple (pair ou impair) du quart ou de la moitié de la longueur d'onde.
Egalement, même si dans les différents modes de réalisation présentés précédemment, l'épaisseur de fluide de refroidissement était fixe, celle-ci pourrait être variable, par exemple en utilisant un matériau flexible et compressible pour constituer la paroi interne. Dans ce cas, une variation de la pression de fluide de refroidissement pourrait permettre de faire varier l'épaisseur de la couche de fluide de refroidissement. Par exemple une augmentation (respectivement une diminution) de la pression de fluide de refroidissement permet d'augmenter (respectivement diminuer) l'épaisseur de la couche de fluide de refroidissement. Cette variation de la pression de fluide de refroidissement peut avantageusement être contrôlée par des moyens prévus dans le générateur 6 et l'unité de commande 5. Notamment le générateur 6 peut comporter un (ou plusieurs) coupleur qui permet de mesurer la tension, le courant ou la puissance transmise ou réfléchie par la sonde. Lorsque l'épaisseur de la couche de fluide varie, l'onde secondaire est plus ou moins réfléchie vers le transducteur, ce qui se traduit par des variations dans les mesures effectuées par le coupleur. Ces mesures sont interprétées dans l'unité de commande 5 et renseignent l'utilisateur sur l'état de la sonde, par exemple sur la pression de son liquide de refroidissement.
Par conséquent, toutes les modifications de ce type sont destinées à être incorporées à l'intérieur de la portée des revendications jointes.

**Revendications**

**1.** Sonde ultrasonore pour le chauffage, par voie interstitielle ou au travers d'un cathéter, d'un milieu cible absorbant les ultrasons, la sonde comprenant :

- au moins un transducteur piézoélectrique (21) comportant une face avant (212) destinée à être positionnée en regard du milieu cible et une face arrière (211) opposée à la face avant (212), le transducteur étant apte à émettre au moins une onde primaire émanant de sa face avant et au moins une onde secondaire émanant de sa face arrière,
- un réflecteur (24) s'étendant en regard de la face arrière (211) du transducteur (21), le réflecteur (24) étant adapté pour réfléchir l'onde secondaire émise par le transducteur (21),
- une couche de fluide de refroidissement (25) entre le transducteur (21) et le réflecteur (24),

**caractérisée en ce que** l'épaisseur (e2) de la couche de fluide de refroidissement (25) est adaptée en fonction :

• du matériau constituant le fluide de refroidissement, et

• de l'épaisseur (e1) et d'un matériau constituant le réflecteur (24), et
• de la fréquence nominale du transducteur (21),

de sorte qu'une onde ultrasonore secondaire réfléchie par le réflecteur (24) et se propageant vers la face avant (212) interfère de façon constructive avec une onde ultrasonore primaire émanant de la face avant (212) du transducteur (21).

2. Sonde ultrasonore selon la revendication 1, dans laquelle le transducteur (21) est alimenté électriquement par un signal électrique d'excitation induisant l'émission par ledit transducteur (21) d'ondes ultrasonores à une fréquence nominale comprise entre 3 et 10 MHz, le réflecteur (24) étant adapté pour réfléchir au moins 80% de l'énergie acoustique de l'onde secondaire émise par le transducteur (21) à la fréquence nominale du transducteur, et le fluide de refroidissement ayant un coefficient d'atténuation acoustique inférieur à 1dB/cm à la fréquence nominale du transducteur, de préférence inférieur à 0.1 dB/cm à la fréquence nominale du transducteur.

3. Sonde selon l'une quelconque des revendications 1 ou 2, dans laquelle en fonction de l'épaisseur (e1) et du matériau constituant le réflecteur (24), l'épaisseur (e2) de la couche de fluide de refroidissement (25) est :

• soit égale à un multiple impair du quart de la longueur d'onde de l'onde ultrasonore secondaire dans le matériau constitutif du fluide de refroidissement,
• soit égale à un multiple entier de la moitié de la longueur d'onde de l'onde ultrasonore secondaire dans le matériau constitutif du fluide de refroidissement.

4. Sonde selon l'une quelconque des revendications 1 à 3, dans laquelle :

- l'impédance acoustique du matériau constituant le réflecteur (24) est supérieure à $10^7$ kg/(m²s), et
- l'épaisseur (e2) de la couche de fluide de refroidissement (25) est égale à un multiple impair du quart de la longueur d'onde de l'onde ultrasonore secondaire dans le matériau constitutif du fluide de refroidissement.

5. Sonde selon la revendication 4, dans laquelle l'épaisseur (e1) du réflecteur (24) est supérieure ou égale à 20 $\mu$m, préférentiellement supérieure à 30 $\mu$m, et encore plus préférentiellement supérieure à 40 $\mu$m.

6. Sonde selon l'une quelconque des revendications 1 à 3, dans laquelle le réflecteur (24) comprend une paroi interne incluant une première face (241) en regard de la face arrière (211) du transducteur (21) et une deuxième face (242) opposée, la deuxième face (242) étant en contact avec de l'air.

7. Sonde selon la revendication 6, dans laquelle :

- l'impédance acoustique du matériau constituant la paroi interne est comprise entre $1 \times 10^6$ kg/(m²s) et $10 \times 10^6$ kg/(m²s),
- l'épaisseur (e1) de la paroi interne est égale à un multiple impair du quart de la longueur d'onde de l'onde ultrasonore secondaire dans le matériau constituant la paroi interne, et
- l'épaisseur (e2) de la couche de fluide de refroidissement (25) est égale à un multiple impair du quart de la longueur d'onde de l'onde ultrasonore secondaire dans le fluide de refroidissement.

8. Sonde selon l'une quelconque des revendications 6 ou 7, dans laquelle :

- l'impédance acoustique du matériau constituant la paroi interne est comprise entre $1 \times 10^6$ kg/(m²s) et $10 \times 10^6$ kg/(m²s), et
- l'épaisseur (e1) de la paroi interne et l'épaisseur (e2) de la couche de fluide de refroidissement ne sont pas comprises dans des plages de plus ou moins 25% autour d'un multiple entier de la moitié de la longueur d'onde de l'onde ultrasonore secondaire dans le matériau constituant la paroi interne.

9. Sonde selon la revendication 6, dans laquelle :

- l'impédance acoustique du matériau constituant la paroi interne est comprise entre $1 \times 10^6$ kg/(m²s) et $10 \times 10^6$ kg/(m²s),
- l'épaisseur (e1) de la paroi interne est égale à un multiple entier de la moitié de la longueur d'onde de l'onde ultrasonore secondaire dans le matériau constituant la paroi interne,

- l'épaisseur (e2) de la couche de fluide de refroidissement (25) est égale à un multiple entier de la moitié de la longueur d'onde de l'onde ultrasonore secondaire dans le fluide de refroidissement.

10. Sonde selon l'une quelconque des revendications 6 ou 9, dans laquelle :

- l'impédance acoustique du matériau constituant la paroi interne est comprise entre $1 \times 10^6$ kg/(m²s) et $10 \times 10^6$ kg/(m²s), et
- l'épaisseur (e1) de la paroi interne et l'épaisseur (e2) de la couche de fluide de refroidissement ne sont pas comprises dans des plages de plus ou moins 25% autour d'un multiple impair du quart de la longueur d'onde de l'onde ultrasonore secondaire dans le matériau constituant la paroi interne.

11. Sonde selon l'une quelconque des revendications 6 à 10, dans laquelle la paroi interne est constituée dans un matériau flexible et compressible.

12. Sonde selon la revendication 11, laquelle étant connectée à un système de refroidissement (7) pour l'alimentation de la sonde en fluide de refroidissement, ledit système de refroidissement (7) étant reliée à une unité de contrôle (5) apte à faire varier la pression de fluide de refroidissement pour augmenter ou diminuer l'épaisseur de la couche de fluide de refroidissement.

13. Sonde selon l'une quelconque des revendications 1 à 12, laquelle comprend en outre une couche d'adaptation (29) - par exemple composée de parylène - sur la face avant (212) du transducteur (21), ladite couche d'adaptation (29) ayant une épaisseur égale à un multiple du quart de la longueur d'onde de l'onde ultrasonore dans le matériau constitutif de ladite couche d'adaptation - par exemple sensiblement égale à 80 $\mu$m pour une fréquence centrale de résonance égale à 6 MHz.

14. Sonde selon l'une quelconque des revendications 1 à 12, dans laquelle le transducteur est composé d'éléments piézoélectriques (21a-21d) obtenus en sectorisant un transducteur tubulaire monobloc à l'aide d'un laser haute puissance ou d'un fraisage mécanique permettant de former des rainures longitudinales, la largeur des rainures étant inférieure à la longueur d'onde de l'onde primaire, et la somme de la largeur des rainures étant inférieure au huitième du périmètre extérieur de la sonde.

15. Sonde selon la revendication 14, laquelle comprend en outre un ballonnet (31) à volume variable s'étendant sur la face extérieure du transducteur (21), le ballonnet (31) étant connecté à des moyens d'alimentation en fluide permettant de faire varier le volume du ballonnet (31) entre :

- une conformation rétractée où le volume du ballonnet est minimale, et
- une conformation déployée où le volume du ballonnet est maximale.

**Patentansprüche**

1. Ultraschallsonde zur Erwärmung eines Zielmediums, das Ultraschallwellen absorbiert, interstitiell oder über einen Katheter, wobei die Sonde umfasst:

- mindestens einen piezoelektrischen Wandler (21), der eine Vorderseite (212), die dazu bestimmt ist, dem Zielmedium gegenüberliegend positioniert zu werden, und eine Hinterseite (211) umfasst, die der Vorderseite (212) entgegengesetzt ist, wobei der Wandler dazu geeignet ist, mindestens eine Primärwelle, die von seiner Vorderseite ausgeht, und mindestens eine Sekundärwelle auszustrahlen, die von seiner Hinterseite ausgeht,
- einen Reflektor (24), der sich der Hinterseite (211) des Wandlers (21) gegenüberliegend erstreckt, wobei der Reflektor (24) dazu geeignet ist, die von dem Wandler (21) ausgestrahlte Sekundärwelle zu reflektieren,
- eine Kühlmittelschicht (25) zwischen dem Wandler (21) und dem Reflektor (24),

**dadurch gekennzeichnet, dass** die Dicke (e2) der Kühlmittelschicht (25) in Abhängigkeit von Folgendem angepasst ist:

- dem Material, aus dem das Kühlmittel besteht, und
- der Dicke (e1) und einem Material, aus dem der Reflektor (24) besteht, und
- der Nennfrequenz des Wandlers (21),

derart, dass eine Sekundärultraschallwelle, die von dem Reflektor (24) reflektiert wird und sich hin zu der Vorderseite (212) ausbreitet, sich auf konstruktive Weise mit einer Primärultraschallwelle überlagert, die von der Vorderseite (212) des Wandlers (21) ausgeht.

2. Ultraschallsonde nach Anspruch 1, wobei der Wandler (21) elektrisch durch ein elektrisches Erregungssignal versorgt wird, das das Ausstrahlen von Ultraschallwellen mit einer Nennfrequenz von zwischen 3 und 10 MHz durch den Wandler (21) bewirkt, wobei der Reflektor (24) dazu geeignet ist, mindestens 80 % der akustischen Energie der von dem Wandler (21) bei der Nennfrequenz des Wandlers ausgestrahlten Sekundärwelle zu reflektieren, und das Kühlmittel einen akustischen Dämpfungskoeffizienten von kleiner als 1 dB/cm bei der Nennfrequenz des Wandlers, vorzugsweise kleiner als 0,1 dB/cm bei der Nennfrequenz des Wandlers, aufweist.

3. Sonde nach einem der Ansprüche 1 oder 2, wobei in Abhängigkeit von der Dicke (e1) und dem Material, aus dem der Reflektor (24) besteht, die Dicke (e2) der Kühlmittelschicht (25) wie folgt ist:

   • entweder gleich einem ungeraden Vielfachen von einem Viertel der Wellenlänge der Sekundärultraschallwelle in dem Material, aus dem das Kühlmittel besteht,
   • oder gleich einem ganzzahligen Vielfachen der Hälfte der Wellenlänge der Sekundärultraschallwelle in dem Material, aus dem das Kühlmittel besteht.

4. Sonde nach einem der Ansprüche 1 bis 3, wobei:

   - die akustische Impedanz des Materials, aus dem der Reflektor (24) besteht, größer als $10^7$ kg/(m²s) ist, und
   - die Dicke (e2) der Kühlmittelschicht (25) gleich einem ungeraden Vielfachen von einem Viertel der Wellenlänge der Sekundärultraschallwelle in dem Material ist, aus dem das Kühlmittel besteht.

5. Sonde nach Anspruch 4, wobei die Dicke (e1) des Reflektors (24) größer oder gleich 20 $\mu$m, vorzugsweise größer als 30 $\mu$m und, und noch mehr zu bevorzugen, größer als 40 $\mu$m ist.

6. Sonde nach einem der Ansprüche 1 bis 3, wobei der Reflektor (24) eine Innenwand umfasst, die eine erste Seite (241), die der Hinterseite (211) des Wandlers (21) gegenüberliegt, und eine zweite, entgegengesetzte Seite (242) einschließt, wobei die zweite Seite (242) mit Luft in Kontakt ist.

7. Sonde nach Anspruch 6, wobei:

   - die akustische Impedanz des Materials, aus dem die Innenwand besteht, zwischen $1{\times}10^6$ kg/(m²s) und $10{\times}10^6$ kg/(m²s) beträgt,
   - die Dicke (e1) der Innenwand gleich einem ungeraden Vielfachen von einem Viertel der Wellenlänge der Sekundärultraschallwelle in dem Material ist, aus dem die Innenwand besteht, und
   - die Dicke (e2) der Kühlmittelschicht (25) gleich einem ungeraden Vielfachen von einem Viertel der Wellenlänge der Sekundärwellenlänge in dem Kühlmittel ist.

8. Sonde nach einem der Ansprüche 6 oder 7, wobei:

   - die akustische Impedanz des Materials, aus dem die Innenwand besteht, zwischen $1{\times}10^6$ kg/(m²s) und $10{\times}10^6$ kg/(m²s) beträgt, und
   - die Dicke (e1) der Innenwand und die Dicke (e2) der Kühlmittelschicht nicht in den Bereichen von plus/minus 25 % um ein ganzzahliges Vielfaches der Hälfte der Wellenlänge der Sekundärultraschallwelle in dem Material enthalten sind, aus dem die Innenwand besteht.

9. Sonde nach Anspruch 6, wobei:

   - die akustische Impedanz des Materials, aus dem die Innenwand besteht, zwischen $1{\times}10^6$ kg/(m²s) und $10{\times}10^6$ kg/(m²s) beträgt,
   - die Dicke (e1) der Innenwand gleich einem ganzzahligen Vielfachen der Hälfte der Wellenlänge der Sekundärultraschallwelle in dem Material ist, aus dem die Innenwand besteht,
   - die Dicke (e2) der Kühlmittelschicht (25) gleich einem ganzzahligen Vielfachen der Hälfte der Wellenlänge der Sekundärultraschallwelle in dem Kühlmittel ist.

10. Sonde nach einem der Ansprüche 6 oder 9, wobei:

- die akustische Impedanz des Materials, aus dem die Innenwand besteht, zwischen $1 \times 10^6$ kg/(m²s) und $10 \times 10^6$ kg/(m²s) beträgt, und
- die Dicke (e1) der Innenwand und die Dicke (e2) der Kühlmittelschicht nicht in den Bereichen von plus/minus 25 % um ein ungerades Vielfaches von einem Viertel der Wellenlänge der Sekundärultraschallwelle in dem Material enthalten sind, aus dem die Innenwand besteht.

11. Sonde nach einem der Ansprüche 6 bis 10, wobei die Innenwand aus einem biegsamen und zusammendrückbaren Material besteht.

12. Sonde nach Anspruch 11, die mit einem Kühlsystem (7) zur Versorgung der Sonde mit Kühlmittel verbunden ist, wobei das Kühlsystem (7) mit einer Steuereinheit (5) verbunden ist, die geeignet ist, den Druck des Kühlmittels zu verändern, um die Dicke der Kühlmittelschicht zu erhöhen oder zu vermindern.

13. Sonde nach einem der Ansprüche 1 bis 12, die ferner eine Anpassungsschicht (29) - die zum Beispiel aus Parylen besteht - auf der Vorderseite (212) des Wandlers (21) umfasst, wobei die Anpassungsschicht (29) eine Dicke von gleich einem Vielfachen von einem Viertel der Wellenlänge der Ultraschallwelle in dem Material aufweist, aus dem die Anpassungsschicht besteht - zum Beispiel im Wesentlichen von gleich 80 μm für eine Resonanzmittenfrequenz von gleich 6 MHz.

14. Sonde nach einem der Ansprüche 1 bis 12, wobei der Wandler aus piezoelektrischen Elementen (21a bis 21d) besteht, die durch Unterteilen eines einstückigen rohrförmigen Wandlers mittels eines Hochleistungslasers oder eines mechanischen Fräsens, das das Bilden von Längsrillen ermöglicht, erhalten werden, wobei die Breite der Rillen kleiner als die Wellenlänge der Primärwelle ist und die Summe der Breite der Rillen kleiner als ein Achtel des Außenumfangs der Sonde ist.

15. Sonde nach Anspruch 14, die ferner einen Ballon (31) mit variablem Volumen umfasst, der sich auf der Außenseite des Wandlers (21) erstreckt, wobei der Ballon (31) mit Fluidversorgungsmitteln verbunden ist, die das Ändern des Volumens des Ballons (31) ermöglichen zwischen:

- einer zurückgezogenen Ausgestaltung, in der das Volumen des Ballons minimal ist, und
- einer entfalteten Ausgestaltung, in der das Volumen des Ballons maximal ist.

**Claims**

1. An ultrasonic probe for heating, interstitially or through a catheter, a target medium which absorbs ultrasounds, the probe comprising:

- at least one piezoelectric transducer (21) including a front face (212) intended to be positioned facing the target medium and a rear face (211) opposite to the front face (212), the transducer being able to emit at least one primary wave emanating from its front face and at least one secondary wave emanating from its rear face,
- a reflector (24) extending facing the rear face (211) of the transducer (21), the reflector (24) being adapted to reflect the secondary wave emitted by the transducer (21),
- a cooling fluid layer (25) between the transducer (21) and the reflector (24),

**characterized in that** the thickness (e2) of the cooling fluid layer (25) is adapted according to:

• the material constituting the cooling fluid, and
• the thickness (e1) and a material constituting the reflector (24), and
• the nominal frequency of the transducer (21),

so that a secondary ultrasonic wave reflected by the reflector (24) and propagating towards the front face (212) constructively interferes with a primary ultrasonic wave emanating from the front face (212) of the transducer (21).

2. The ultrasonic probe according to claim 1, wherein the transducer (21) is electrically powered by an electrical excitation signal inducing the emission by said transducer (21) of ultrasonic waves at a nominal frequency comprised between 3 and 10 MHz, the reflector (24) being adapted to reflect at least 80% of the acoustic energy of the secondary wave emitted by the transducer (21) at the nominal frequency of the transducer, and the cooling fluid having an acoustic attenuation coefficient less than 1dB/cm at the nominal frequency of the transducer, preferably less than 0.1 dB/cm at the nominal frequency of the transducer.

3. The ultrasonic probe according to any one of claims 1 or 2, wherein depending on the thickness (e1) and the material constituting the reflector (24), the thickness (e2) of the cooling fluid layer (25) is:

• either equal to an odd multiple of the quarter of the wavelength of the secondary ultrasonic wave in the material constituting the cooling fluid,
• or equal to an integer multiple of half the wavelength of the secondary ultrasonic wave in the material constituting the cooling fluid.

4. The ultrasonic probe according to any one of claims 1 to 3, wherein:

- the acoustic impedance of the material constituting the reflector (24) is greater than $10^7$ kg/(m$^2$s), and
- the thickness (e2) of the cooling fluid layer (25) is equal to an odd multiple of the quarter of the wavelength of the secondary ultrasonic wave in the material constituting the cooling fluid.

5. The ultrasonic probe according to claim 4, wherein the thickness (e1) of the reflector (24) is greater than or equal to 20 $\mu$m, preferably greater than 30 $\mu$m, and even more preferably greater than 40 $\mu$m.

6. The ultrasonic probe according to any one of claims 1 to 3, wherein the reflector (24) comprises an inner wall including a first face (241) facing the rear face (211) of the transducer (21) and a second opposite face (242), the second face (242) being in contact with air.

7. The ultrasonic probe according to claim 6, wherein:

- the acoustic impedance of the material constituting the inner wall is comprised between $1 \times 10^6$ kg/(m$^2$s) and $10 \times 10^6$ kg/(m$^2$s),
- the thickness (e1) of the inner wall is equal to an odd multiple of the quarter of the wavelength of the secondary ultrasonic wave in the material constituting the inner wall, and
- the thickness (e2) of the cooling fluid layer (25) is equal to an odd multiple of the quarter of the wavelength of the secondary ultrasonic wave in the cooling fluid.

8. The ultrasonic probe according to any one of claims 6 or 7, wherein:

- the acoustic impedance of the material constituting the inner wall is comprised between $1 \times 10^6$ kg/(m$^2$s) and $10 \times 10^6$ kg/(m$^2$s), and
- the thickness (e1) of the inner wall and the thickness (e2) of the cooling fluid layer are not within ranges of more or less 25% around an integer multiple of half the wavelength of the secondary ultrasonic wave in the material constituting the inner wall.

9. The ultrasonic probe according to claim 6, wherein:

- the acoustic impedance of the material constituting the inner wall is comprised between $1 \times 10^6$ kg/(m$^2$s) and $10 \times 10^6$ kg/(m$^2$s),
- the thickness (e1) of the inner wall is equal to an integer multiple of half the wavelength of the secondary ultrasonic wave in the material constituting the inner wall,
- the thickness (e2) of the cooling fluid layer (25) is equal to an integer multiple of half the wavelength of the secondary ultrasonic wave in the cooling fluid.

10. The ultrasonic probe according to any one of claims 6 or 9, wherein:

- the acoustic impedance of the material constituting the inner wall is comprised between $1 \times 10^6$ kg/(m$^2$s) and $10 \times 10^6$ kg/(m$^2$s), and
- the thickness (e1) of the inner wall and the thickness (e2) of the cooling fluid layer are not within ranges of more or less 25% around an odd multiple of the quarter of the wavelength of the secondary ultrasonic wave in the material constituting the inner wall.

11. The ultrasonic probe according to any one of claims 6 to 10, wherein the inner wall is made of a flexible and compressible material.

12. The ultrasonic probe according to claim 11, which being connected to a cooling system (7) for supplying the probe with cooling fluid,
said cooling system (7) being connected to a monitoring unit (5) able to vary the cooling fluid pressure to increase or decrease the thickness of the cooling fluid layer.

13. The ultrasonic probe according to any one of claims 1 to 12, which further comprises an adaptation layer (29) - for example composed of parylene - on the front face (212) of the transducer (21), said adaptation layer (29) having a thickness equal to a multiple of the quarter of the wavelength of the ultrasonic wave in the material constituting said adaptation layer - for example substantially equal to 80 $\mu$m for a central resonant frequency equal to 6 MHz.

14. The ultrasonic probe according to any one of claims 1 to 12, wherein the transducer is composed of piezoelectric elements (21a-21d) obtained by sectoring a one-piece tubular transducer using a high-power laser or a mechanical milling that allows forming longitudinal grooves, the width of the grooves being less than the wavelength of the primary wave, and the sum of the width of the grooves being less than one-eighth of the external perimeter of the probe.

15. The ultrasonic probe according to claim 14, which further comprises a balloon (31) of variable volume extending on the external face of the transducer (21), the balloon (31) being connected to fluid supply means that allow varying the volume of the balloon (31) between:

    - a retracted conformation where the volume of the balloon is minimal, and
    - a deployed conformation where the volume of the balloon is maximal.

**FIG.1**

| UNITE DE COMMANDE | GENERATEUR | SONDE |
|---|---|---|

SYSTEME DE REFROIDISSEMENT

**FIG.2**

**FIG.3**

**FIG.4**

**FIG.5**

Epaisseur (en µm)

**FIG.6A**

**FIG.6B**

**FIG.6C**

RECEPTION PARAMETRES ~ 100

DIMENSIONNEMENT ~ 200

ASSEMBLAGE ~ 300

**FIG.7**

**EP 3 442 442 B1**